(19) Europäisches Patentamt
European Patent Office
Office européen des brevets

(11) **EP 3 788 637 B1**

(12) **FASCICULE DE BREVET EUROPEEN**

(45) Date de publication et mention
de la délivrance du brevet:
**04.06.2025 Bulletin 2025/23**

(21) Numéro de dépôt: **19729339.2**

(22) Date de dépôt: **30.04.2019**

(51) Classification Internationale des Brevets (IPC):
**G16H 40/63** $^{(2018.01)}$ **G16H 50/20** $^{(2018.01)}$
**G16H 50/30** $^{(2018.01)}$

(52) Classification Coopérative des Brevets (CPC):
**G16H 40/63; G16H 50/20**

(86) Numéro de dépôt international:
**PCT/FR2019/051010**

(87) Numéro de publication internationale:
**WO 2019/211561 (07.11.2019 Gazette 2019/45)**

(54) **PROCÉDÉ DE TRAITEMENT D'UN SIGNAL ACCÉLÉROMÉTRIQUE**

VERFAHREN ZUR VERARBEITUNG EINES BESCHLEUNIGUNGSSIGNALS

METHOD FOR PROCESSING AN ACCELEROMETRIC SIGNAL

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité: **02.05.2018 FR 1853796**

(43) Date de publication de la demande:
**10.03.2021 Bulletin 2021/10**

(73) Titulaire: **Université Grenoble Alpes
38400 Saint-Martin-d'Hères (FR)**

(72) Inventeurs:
• **GERARD, Grégoire
69160 TASSIN LA DEMI LUNE (FR)**
• **GUMERY, Pierre-Yves
38000 GRENOBLE (FR)**
• **COLAS, Damien
69300 CALUIRE-ET-CUIRE (FR)**
• **BRICOUT, Aurélien
69002 LYON (FR)**

(74) Mandataire: **INNOV-GROUP
209 Avenue Berthelot
69007 Lyon (FR)**

(56) Documents cités:
**EP-A1- 2 974 648     WO-A1-2018/033889
WO-A1-2018/178569**

• **ATENA ROSHAN FEKR ET AL: "Design of an e-Health Respiration and Body Posture Monitoring System and Its Application for Rib Cage and Abdomen Synchrony Analysis", 2014 IEEE INTERNATIONAL CONFERENCE ON BIOINFORMATICS AND BIOENGINEERING, 9 February 2015 (2015-02-09), pages 141 - 148, XP055535504, ISBN: 978-1-4799-7502-0, DOI: 10.1109/BIBE.2014.67**
• **ATENA ROSHAN FEKR ET AL: "Respiration Disorders Classification With Informative Features for m-Health Applications", IEEE JOURNAL OF BIOMEDICAL AND HEALTH INFORMATICS, vol. 20, no. 3, 21 July 2015 (2015-07-21), Piscataway, NJ, USA, pages 733 - 747, XP055535505, ISSN: 2168-2194, DOI: 10.1109/JBHI.2015.2458965**

EP 3 788 637 B1

**Description**

DOMAINE DE L'INVENTION

La présente invention concerne le domaine du suivi respiratoire (indistinctement ventilatoire) d'un individu.

Plus précisément, elle se rapporte dans le domaine du bien être et de la santé, à la mesure et à la caractérisation de la fonction ventilatoire d'un individu grâce à un ensemble d'accéléromètres en réseau.

La présente invention permet le recueil de données relatives à la respiration d'un individu. L'analyse ultérieure de ces données peut être utile par exemple pour la caractérisation d'une dynamique ventilatoire ou d'un diagnostic médical.

Des méthodes d'étude de la respiration à l'aide de deux accéléromètres tridimensionnels, un en position thoracique et un en position abdominale, sont connu de ATENA ROSHAN FEKR ET AL: "Respiration Disorders Classification With Informative Features for m-Health Applications", IEEE JOURNAL OF BIOMEDICAL AND HEALTH INFORMATICS, vol. 20, no. 3, 21 juillet 2015 (2015-07-21), pages 733-747, Piscataway, NJ, USA.

La présente invention trouve des applications par exemple dans :

- l'analyse du sommeil et la recherche de troubles respiratoires du sommeil,
- le sport et l'exploration des fonctions respiratoires, en particulier au regard des performances sportives aérobies de l'individu, mais également pour l'entraînement à la plongée en apnée,
- le bien-être du grand public, par exemple pour l'évaluation de la dynamique respiratoire en position statique,
- le suivi de la dynamique respiratoire dans les pratiques de relaxation comme le yoga,
- l'analyse de la réponse d'un individu à un stress donné, par exemple pour une campagne vidéo, un film, une musique, etc.
- le domaine de la recherche clinique et pré-clinique pour l'évaluation et la validation de l'effet de certains médicaments sur les fonctions respiratoires,
- le domaine de médecine générale et hospitalière pour le dépistage rapide de pathologies respiratoire.

A l'heure actuelle, il existe des bandes de pléthysmographie respiratoire qui se fixent autour du tronc d'un individu. Toutefois, de telles bandes sont peu pratiques à l'utilisation car elles peuvent bouger et/ou se détériorer pendant leur utilisation, ce qui rend alors inexploitables leurs données, et leur fragilité intrinsèque rend leur utilisation délicate.

Il apparaît donc souhaitable d'améliorer cette solution existante.

RESUME DE L'INVENTION

Plus précisément, l'invention concerne un procédé de traitement de signal, selon les caracteristiques de la revendication 1. Des autres implémentations avantageuses se trouvent dans les revendications dépendantes.

Selon l'invention, le couple d'accéléromètres permet à lui seul, de reconstituer l'équivalent d'une Pléthysmographie Respiratoire par Inductance (PRI). En ce sens, l'invention peut être considérée comme un pléthysmographe respiratoire virtuel car composé au minimum uniquement de deux accéléromètres, un posé sur le thorax du sujet et l'autre sur l'abdomen du sujet.

L'invention trouve un intérêt notamment dans la miniaturisation du système de mesure, moins complexe, moins coûteux, non intrusif et moins invasif que les systèmes de référence en hôpital, et dans sa capacité d'accéder à une mesure fiable, robuste et rapide.

D'autres caractéristiques et avantages de la présente invention apparaîtront plus clairement à la lecture de la description suivante donnée à titre d'exemple illustratif et non limitatif et faite en référence aux figures annexées.

DESCRIPTIF DES DESSINS

- la figure 1 illustre un ensemble 6 de signaux bruts issus selon les 3 voies d'un accéléromètre thoracique et d'un accéléromètre abdominal, ainsi qu'un signal de référence obtenu par une bande pléthysmographique abdominale et un signal de référence obtenu par une bande pléthysmographique abdominale,
- la figure 2 illustre de manière synchrone un signal PHI expérimental et un signal PRI de référence, obtenus selon les signaux de la figure 1,
- la figure 3 illustre la concordance entre les deux signaux PHI et PRI de la figure 2 selon un graphique de Bland-Altman,
- la figure 4 illustre de manière synchrone un signal dPHI, dérivé du signal PHI de la figure 2, un signal dPRI, dérivé du signal PRI de la figure 2, et un signal FLOW de référence,
- la figure 5 illustre de manière synchrone un signal sPHI et un signal sPRI,

- la figure 6 illustre le calcul d'un index de caractérisation de la périodicité d'un effort ventilatoire thoracique,
- la figure 7 illustre le calcul d'un index de caractérisation de la périodicité d'un autre effort ventilatoire thoracique,
- la figure 8A illustre un mode de réalisation d'un dispositif de mesure selon l'invention
- la figure 8B illustre un autre mode de réalisation d'un dispositif de mesure selon l'invention

## DESCRIPTION DETAILLEE

Par concision, on entend par :

- « accéléromètre thoracique » ou « premier accéléromètre », un premier ensemble d'au moins un accéléromètre tridimensionnel positionné en position thoracique du sujet,
- « accéléromètre abdominal » ou « deuxième accéléromètre » : un deuxième ensemble d'au moins un accéléromètre tridimensionnel positionné en position abdominal du sujet,
- « Individu », « patient » ou « sujet », toute personne physique, sain ou atteint d'une pathologie quelconque,
- « signaux » ou données, les valeurs mesurées selon chaque axe du premier et du deuxième accéléromètre,
- « compartiment » le thorax ou l'abdomen.

### Dispositif de mesure

On prévoit un dispositif de mesure unique et compact comportant au moins deux accéléromètres configurés pour mesurer l'accélération selon trois axes.

En particulier, on prévoit un premier ensemble d'au moins un premier accéléromètre, destiné à être placé en position thoracique de l'individu, de préférence en position xiphoïde.

On prévoit également un deuxième ensemble d'au moins un accéléromètre, destiné à être placé en position abdominale dudit individu.

Chaque ensemble d'au moins un accéléromètre peut comprendre une pluralité d'accéléromètres par exemple pour des raisons de redondance et robustesse.

Par concision, le premier ensemble d'au moins un accéléromètre est appelé premier accéléromètre 110, et le deuxième ensemble d'au moins un accéléromètre est appelé deuxième accéléromètre 120.

En l'espèce, un seul accéléromètre par ensemble peut suffire, c'est à dire un unique premier accéléromètre placé en position thoracique et un unique deuxième accéléromètre en position abdominale.

Chaque accéléromètre émet un signal respectif comprenant un ensemble de données et appelé « signal initial », qui correspond à l'évolution des accélérations (en g) en fonction du temps. Par concision, on entend indistinctement « signal » et « données ».

De préférence, on prévoit que le dispositif de mesure comprend des moyens d'activation / désactivation des accéléromètres, par exemple sous forme d'interrupteur de commande d'activation, analogique ou numérique, éventuellement sans fil.

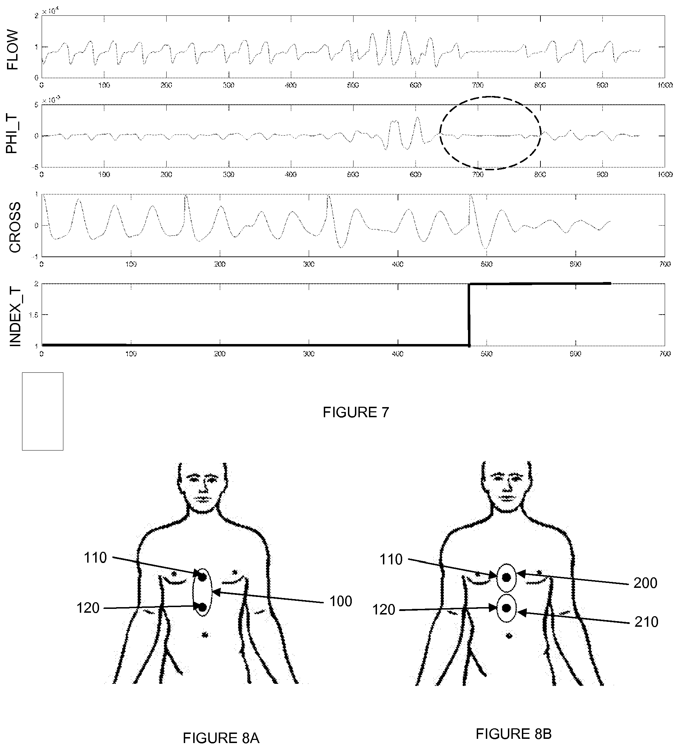

Dans une première variante, on prévoit que le premier accéléromètre et le deuxième accéléromètre sont solidaires d'un unique support 100 (figure 8A).

Dans une seconde variante, on prévoit que le premier accéléromètre et le deuxième accéléromètre sont solidaires d'un support respectif 200, 210 (figure 8B).

Quelle que soit la variante, chaque support comprend une face supérieure et une face inférieure. La face supérieure supporte au moins un accéléromètre et la face inférieure, destinée à être au contact de l'individu, est typiquement enduite d'adhésif (colle), de préférence repositionnable. L'avantage de l'aspect repositionnable est que l'individu peut retirer le dispositif de mesure au réveil, après une douche, etc. et réutiliser le même dispositif de mesure ensuite, et ce sur plusieurs jours consécutifs. Le dispositif est par exemple alimenté par une batterie.

Le premier accéléromètre et le deuxième accéléromètre peuvent être identiques. En l'espèce, le premier accéléromètre et le deuxième accéléromètre sont des accéléromètres trois axes standard.

Les données issues de chaque capteur sont enregistrées dans une mémoire informatique. Elles sont envoyées par voie filaire ou sans fil.

Dans une première variante, la mémoire est en connexion informatique filaire avec le premier et le deuxième accéléromètre. La mémoire peut par exemple être disposée sur le support unique ou l'un quelconque des supports des accéléromètres.

De préférence, on prévoit que la mémoire est en connexion informatique avec un port de communication d'entrée/sortie (I/O), par exemple un port USB, ce qui permet d'exporter les données enregistrées vers un dispositif de traitement des données comprenant un logiciel de traitement des données, typiquement tout objet communicant, c'est-à-dire un dispositif électronique comprenant des moyens de communication avec ou sans fil, un processeur et de préférence un écran d'affichage, par exemple un ordinateur personnel, un *smartphone,* une tablette tactile etc.

On peut aussi prévoir que la mémoire est amovible, par exemple sous forme de support de donnée de type carte mémoire à connexion USB, connectable au premier accéléromètre et au deuxième accéléromètre pour l'enregistrement des données issues de ceux-ci, et connectable ensuite à un ordinateur équipé d'un logiciel de filtrage permettant de filtrer lesdites données.

Dans une deuxième variante, la mémoire est déportée, c'est-à-dire sans connexion filaire entre ladite mémoire et les capteurs. Par exemple, la mémoire est déportée dans le dispositif de traitement des données.

Dans ce cas, on prévoit avantageusement que le dispositif de mesure comprend des moyens de communication avec ledit dispositif de traitement des données, que ce soit une communication filaire ou sans fil.

La mémoire peut donc être embarquée ou déportée, sur un serveur, en particulier d'un système informatique en nuage.

Les accéléromètres sont montés avantageusement sur une carte électronique, comportant une batterie, un processeur (microcontrôleur) et une unité de stockage pouvant stocker les données dans l'optique d'un traitement embarqué. Alternativement, chaque accéléromètre est monté sur une carte électronique indépendante, chacune disposant d'une pile d'alimentation, d'un processeur et d'une unité de stockage. On prévoit que les capteurs optionnels sont solidaires de la carte électronique du premier ensemble d'au moins un accéléromètre La synchronisation des signaux est faite par construction par le microcontrôleur.

De préférence, on prévoit également des moyens de traitement des signaux issus des capteurs.

En l'espèce, les moyens de traitement des signaux comprennent des moyens de filtrage, configurés pour filtrer le signal initial du premier accéléromètre, et configurés pour filtrer le signal initial du deuxième accéléromètre.

De manière similaire à la mémoire, les moyens de filtrage peuvent être intégrés au dispositif de mesure, ou déportés sur le dispositif de traitement des données ou encore sur un serveur en communication avec le dispositif de traitement des données.

Dans ce cas, on prévoit avantageusement que le dispositif de mesure comprend des moyens de communication avec lesdits moyens de traitement des signaux, que ce soit une communication filaire ou sans fil.

Chaque accéléromètre tridimensionnel possède 3 voies (ou axes) orthonormées et appelées respectivement X, Y et Z, dont il conviendra de déterminer la position angulaire par rapport à la gravité.

Par convention, on entend par :

- X1, Y1 et Z1 les 3 voies du premier accéléromètre,
- X2, Y2 et Z2 les 3 voies du deuxième accéléromètre.

A noter qu'une fois le premier accéléromètre et le deuxième accéléromètre positionnés sur un individu, les axes X1, Y1 et Z1 ne sont pas nécessairement respectivement confondus avec les axes X2, Y2 et Z2.

Pour chaque accéléromètre, les données échantillonnées sont enregistrées sous forme de matrice de départ à trois colonnes, une colonne pour l'axe X, une colonne pour l'axe Y et une colonne pour l'axe Z ; la matrice comprenant autant de lignes que de points d'échantillonnage, par exemple, $L=60*T*F$ avec L le nombre total de lignes, T le temps d'échantillonnage en minutes et F la fréquence d'échantillonnage.

On a donc une matrice de départ du compartiment thoracique et une matrice de départ du compartiment abdominal.

La respiration de l'individu génère des mouvements de sa cage thoracique et/ou de son abdomen. Le premier accéléromètre et le deuxième accéléromètre permettent, comme décrit ultérieurement, de reconstruire ces mouvements, notamment par calcul de leur vitesse angulaire respective.

Comme les axes XYZ sont orthonormés, la connaissance de la position de deux axes parmi X, Y et Z par rapport à la gravité permet de reconstruire l'autre. Et lorsqu'il existe une convention de pose déterminée de l'accéléromètre sur l'individu, cela permet de fixer le référentiel individu /accéléromètre, alors dans ce cas la connaissance de la position d'un seul axe parmi X, Y et Z par rapport à la gravité permet de reconstruire les 2 autres. Quand un individu respire, le déplacement de la cage thoracique ou de l'abdomen est très faible. Par conséquent, la vitesse de déplacement, donc l'accélération est négligeable. Le signal issu d'un accéléromètre n'est donc pas réellement lié au déplacement de la cage thoracique ou au déplacement de l'abdomen au premier ordre.

En revanche, quand un individu respire, les accéléromètres (thoracique ou abdominal) subissent une rotation par rapport à la gravité. Quand un accéléromètre pivote, le vecteur gravité pivote également dans le référentiel X1Y1Z1 du premier accéléromètre et dans le référentiel X2Y2Z2 du deuxième accéléromètre.

La présente invention vise à suivre ce pivotement, et à calculer l'évolution de l'orientation du vecteur gravité dans le référentiel X1Y1Z1 ou X2Y2Z2 au cours du temps.

De préférence, on considère que l'individu est globalement statique pendant l'enregistrement des données issues des accéléromètres, de sorte à obtenir une stationnarité des signaux, ce qui permet d'éviter que le mouvement de l'individu ne vienne bruiter les mesures des accéléromètres.

Comme décrit ci-après, le principe de l'invention consiste à mesurer les variations d'inclinaison des accéléromètres par rapport à la gravité en fonction d'un axe de référence à chaque changement de stationnarité du signal, puis à traiter ces signaux pour reconstruire la ventilation de l'individu.

**Prétraitement**

On prévoit une étape de prétraitement consistant à sous-échantillonner les signaux issus de chaque voie des accéléromètres.

De préférence, on prévoit que les signaux issus du premier accéléromètre et les signaux issus du deuxième accéléromètre sont échantillonnés à la même fréquence d'échantillonnage Fe.

De préférence, on prévoit que la fréquence d'échantillonnage Fe est inférieure à une valeur seuil prédéterminée, de sorte à sous-échantillonner lesdits signaux.

Par exemple, Fe = 256 Hz.

De préférence, on prévoit également que la fréquence d'échantillonnage Fe est supérieure à une autre valeur seuil prédéterminée. Par exemple, on prévoit que la Fréquence de Nyquist = Fe/2 est supérieure à la fréquence de coupure haute multipliée par une valeur de référence, en l'espèce 10, de sorte à obtenir une fréquence de Nyquist au moins égale à 8 Hz.

**Filtrage**

Après l'étape de prétraitement, on prévoit une étape de filtrage des signaux.

On prévoit de filtrer chaque voie des accéléromètres par un même filtre. De préférence, on prévoit un filtrage passe-bande. On peut prévoir une fréquence de coupure basse comprise entre 0,01 et 0,1 Hz et de fréquence de coupure haute comprise entre 0,6 et 1 Hz. En l'espèce on prévoit un filtre à réponse impulsionnelle réelle (FIR) passe bande de fréquence de coupure basse égale à 0,05 Hz et de fréquence de coupure haute égale à 0,8 Hz.

**Reconstruction**

Après l'étape de filtrage, on prévoit une étape de reconstruction des efforts respiratoires, c'est à dire des mouvements à l'origine des signaux issus du premier accéléromètre (thoracique) et des signaux issus du deuxième accéléromètre (abdominal), selon l'une au moins des deux variantes ci-dessous.

De préférence, chacune des deux variantes ci-dessous est mise en œuvre après chaque détection de changement de position de l'individu.

**1ère variante : analyse en composantes principales (ACP)**

On peut prévoir une étape d'analyse en composantes principales de chaque matrice de départ, pour chaque compartiment, et réduire ainsi la dimensionnalité de chaque matrice.

En l'espèce, cela permet de passer d'une matrice L*3 à un vecteur L*1.

Pour les données issues d'un accéléromètre donné, une analyse en composantes principales de ces données est une projection dans un nouvel espace, c'est à dire une combinaison linéaire de chaque variable initiale, dans un nouvel espace qui décrit le maximum de variance dans le repère XYZ dudit accéléromètre.

Ainsi, à chaque instant, l'ACP permet de déterminer l'axe X1, Y1 ou Z1 du premier accéléromètre et l'axe X2, Y2 ou Z2 du deuxième accéléromètre qui décrit le maximum de variance (au moins 80%) et qui pivote par rapport à l'axe de la gravité.

Les étapes précédentes ont permis de filtrer (supprimer) les bruits d'origines physiologiques (par exemple l'activité cardiaque) et les artefacts divers (mouvements, bruits, etc...) et conserver l'activité respiratoire sur les signaux accélérométriques, autrement dit sur la mesure du pivotement du premier et du deuxième accéléromètre. Par conséquent, l'axe qui présente le maximum de variabilité est celui qui porte la respiration.

En l'espèce, pour chaque accéléromètre, les données échantillonnées sont enregistrées sous forme de matrice de départ à trois colonnes, une colonne pour l'axe X, une colonne pour l'axe Y et une colonne pour l'axe Z ; la matrice comprenant autant de lignes que de points d'échantillonnage, par exemple, L=60*T*F avec L le nombre total de lignes, T le temps d'échantillonnage en minutes et F la fréquence d'échantillonnage.

L'ACP consiste à transformer la matrice de départ en un vecteur d'arrivée dont chaque coefficient est une combinaison linéaire respective de la ligne de la matrice de départ correspondante, donc des 3 axes de la matrice de départ.

En l'espèce, l'ACP consiste en la mesure des valeurs propres et des vecteurs propres de la matrice de variance-covariance de la matrice de départ. Le vecteur propre dont la valeur propre est la plus élevée est considérée comme la composante principale et servira de vecteur pour projection dans un nouvel espace où chaque coordonnée est une combinaison linéaire de chaque axe (X, Y et Z de départ).

On peut alors calculer le vecteur A_Respi résultant de la multiplication de la matrice de départ A PCA par la composante principale CP :

$$CP = \begin{bmatrix} \alpha \\ \beta \\ \gamma \end{bmatrix}$$

$$A_{PCA} = \begin{bmatrix} X1 & Y1 & C1 \end{bmatrix}$$

$$A_{Respi} = A_{PCA} * CP = \begin{bmatrix} X1 & Y1 & Z1 \end{bmatrix} * \begin{bmatrix} \alpha \\ \beta \\ \gamma \end{bmatrix}$$

Chaque coordonnée du vecteur résultant A_Respi est donc, avec comme exemple à la coordonnée i :

$$A_{Respi}(i) = A_{PCA}(i,:) * CP = \begin{bmatrix} X1(i) & Y1(i) & Z1(i) \end{bmatrix} * \begin{bmatrix} \alpha \\ \beta \\ \gamma \end{bmatrix} = \begin{matrix} X1(i)*\alpha & Y1(i)*\beta & Z1(i)*\gamma \end{matrix}$$

On obtient ainsi :

- un premier vecteur final, résultant d'une analyse en composantes principales sur une première matrice de départ correspondant aux données issues du premier accéléromètre, et
- un deuxième vecteur final, résultant d'une analyse en composantes principales sur une deuxième matrice de départ correspondant aux données issues du deuxième accéléromètre.

Chaque vecteur final est une reconstruction : le premier vecteur final est une reconstruction de l'effort respiratoire du compartiment thoracique et le deuxième vecteur final est une reconstruction de l'effort respiratoire du compartiment abdominale.

Toutefois, l'ACP est une méthode qui est sensible au bruit. Le filtrage selon l'invention précédemment décrit permet de réduire le bruit. Cependant, il peut être avantageux, en outre, de détecter certains événements générateurs de bruit, par exemple un changement de position de l'individu.

A cet effet, on peut prévoir de mesurer l'énergie du signal du premier et/ou du deuxième accéléromètre sur un ensemble de fenêtres temporelles glissantes, de préférence deux fenêtres temporelles adjacentes se recouvrant partiellement.

On prévoit alors une étape de seuillage consistant à comparer l'énergie du signal du premier et/ou du deuxième accéléromètre à une valeur seuil prédéterminée, pour chaque fenêtre temporelle, et si l'énergie du signal est supérieure à la valeur seuil prédéterminée, alors les données de cette fenêtre temporelle ne sont pas prises en compte dans l'ACP (i.e. dans la mesure du vecteur propre servant de base pour la projection dans le nouvel espace).

**Filtrage final**

On peut alors prévoir ensuite une étape de filtrage final du vecteur final.

De préférence, le filtrage final est le même que le filtrage passe bande décrit précédemment.

**2ème variante : méthode angulaire**

Il peut également être avantageux, en remplacement ou en complément de la méthode ACP, de mettre en œuvre une méthode dite angulaire.

Dans cette variante, on vise à calculer l'angle de rotation de chaque accéléromètre par rapport à la gravité à chaque temps de mesure.

A cet effet, on prévoit de mesurer et de normaliser le vecteur accélération, noté a.

En conditions normales, l'utilisateur est statique et les accélérations dues à la respiration sont négligeables. Par conséquent, en conditions normales, le vecteur accélération a est sensiblement égal à l'accélération due à la gravité g.

Quand un accéléromètre pivote, le vecteur gravité pivote également dans le référentiel intrinsèque dudit accéléromètre. Un tel pivotement est du à la respiration et varie donc au cours du temps. Par conséquent, on peut considérer que le mouvement angulaire dudit accéléromètre a lieu selon un seul axe de rotation r.

Il s'agit donc de mesurer le mouvement angulaire de l'axe de rotation r dudit accéléromètre.

L'angle de rotation θt et l'axe de rotation rt du vecteur accélération a entre deux mesures consécutives au temps t-1 et au temps t sont donnés respectivement par le produit scalaire et le produit vectoriel des deux vecteurs suivants :

$$\theta_t = \cos^{-1}(\boldsymbol{a}_t \cdot \boldsymbol{a}_{t-1})$$

$$\boldsymbol{r}_t = \boldsymbol{a}_t \times \boldsymbol{a}_{t-1}$$

La respiration est un mouvement oscillatoire. Par conséquent, l'axe de rotation r du vecteur accélération a s'inverse quand la direction de rotation s'inverse. Il apparaît donc utile de normaliser cette direction de l'axe de rotation r dans un hémisphère prédéterminé, par comparaison à un axe de référence $\mathbf{r}_{ref}$.

On peut ainsi définir :

$$r'_t = \begin{cases} \boldsymbol{r}_t, & \boldsymbol{r}_t \cdot \boldsymbol{r}_{ref} \geq 0 \\ -\boldsymbol{r}_t, & \boldsymbol{r}_t \cdot \boldsymbol{r}_{ref} < 0 \end{cases}$$

De préférence, afin de diminuer l'influence du bruit et de favoriser l'influence des mesures proches du temps t, on prévoit de déterminer $\bar{r}_t$ l'axe de rotation r normalisé moyenné sur une fenêtre de temps de durée W tel que :

$$\bar{r}_t = \text{normalise}\left(\sum_{i=-W/2}^{W/2} H(i)\theta_{t+i} r'_{t+i}\right)$$

Avec H(n) une fenêtre de Hamming. On peut utiliser d'autres fenêtres, par exemple une fenêtre rectangulaire, une fenêtre triangulaire, une fenêtre de Hann, une fenêtre de Blackman, une fenêtre de Kaiser, etc.

De manière similaire, on prévoit de déterminer $\bar{a}_t$ l'accélération moyenne normalisée moyennée sur une fenêtre de temps de durée W, de préférence la même fenêtre temporelle que pour $\bar{r}_t$, tel que :

$$\bar{a}_t = \text{normalise}\left(\sum_{i=-W/2}^{W/2} a_{t+i}\right)$$

On peut alors calculer $\phi_t$ l'angle de rotation dudit accéléromètre tel que :

$$\phi_t = \sin^{-1}\left((\bar{\boldsymbol{a}}_t \times \bar{\boldsymbol{r}}_t) \cdot \boldsymbol{a}_t\right)$$

On peut aussi calculer, si nécessaire, la vitesse angulaire ωt telle que :

$$\omega_t = \frac{d\phi}{dt}$$

Au minimum les valeurs de $\phi_t$ l'angle de rotation sont enregistrées sous forme de vecteur final.

**PRI virtuelle**

Ainsi, quelle que soit la première variante, la deuxième variante ou la combinaison des deux variantes mise en œuvre, on obtient un premier vecteur final qui qualifie l'effort thoracique et un deuxième vecteur final qui qualifie l'effort abdominal, ce qui permet d'obtenir une PRI virtuelle.

Grâce à ce premier vecteur final et ce deuxième vecteur final, on peut qualifier l'effort respiratoire global de l'individu, c'est à dire le volume relatif, ainsi que la dynamique temporelle des efforts de chaque compartiment, donc de qualifier leur cohérence temporelle.

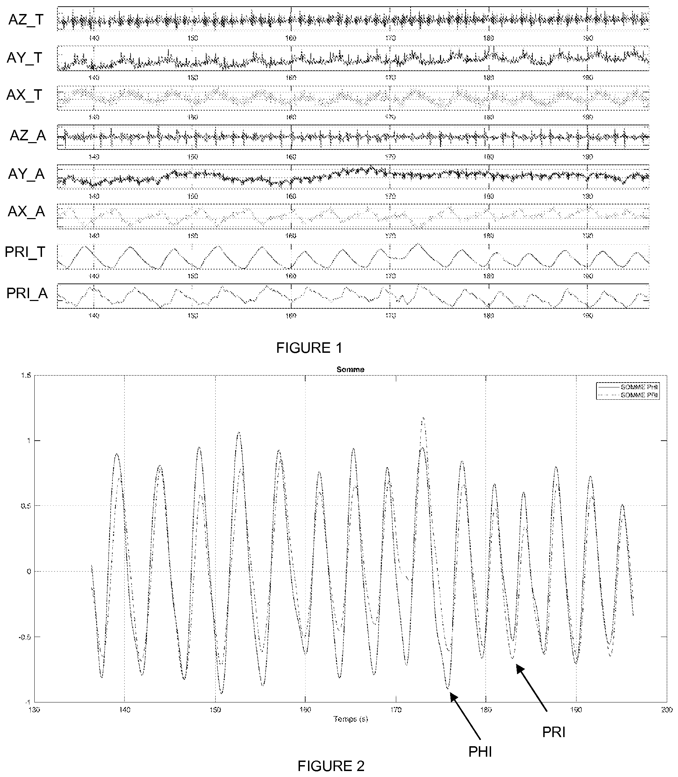

La figure 1 illustre de manière synchrone :

- les données brutes AX_T, AY_T et AZ_T issues du premier accéléromètre selon les axes XYZ dudit premier accéléromètre ;
- les données brutes AX_A, AY_A et AZ_A issues du deuxième accéléromètre, selon les axes XYZ dudit deuxième accéléromètre ;
- les données brutes PRI_T et PRI_A d'une bande de pléthysmographie positionnée respectivement en position thoracique et en position abdominale.

La PRI thoracique (PRI_T) et la PRI abdominale (PRI_A) illustrées en figure 1 sont des variations de surface de section et servent de référence.

La somme de la PRI thoracique (PRI_T) et de la PRI abdominale (PRI_A) est un signal PRI qui donne une image du volume ventilé courant.

La figure 2 illustre la superposition normalisée entre les valeurs -1 et +1 pour un signal PHI expérimental et un signal PRI de référence.

Le signal PRI de référence est la somme des données brutes PRI_T et PRI_A de la figure 1.

Le signal PHI est calculé, en l'espèce selon la méthode angulaire, à partir des données brutes AX_T, AY_T et AZ_T issues du premier accéléromètre et des données brutes AX_A, AY_A et AZ_A issues du deuxième accéléromètre.

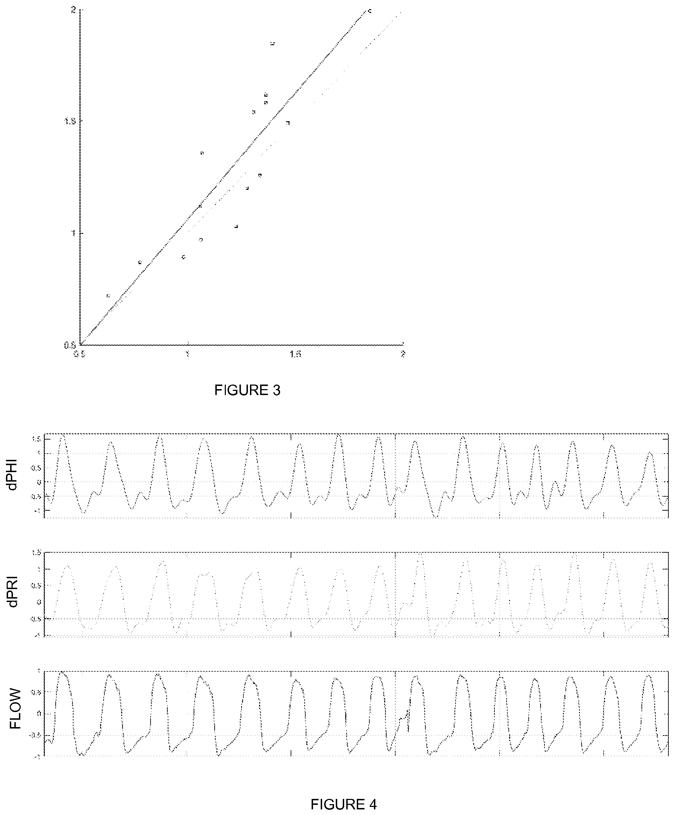

Il est clair que la figure 2 illustre la pertinence de la présente invention. Les deux signaux PHI et PRI sont quasiment toujours en phase. En l'espèce, la concordance entre les deux signaux PHI et PRI peut être établie, par exemple selon un graphique de Bland-Altman, voir figure 3, pour lequel on obtient une valeur de $r^2 = 0.78$.

## Reconstruction d'une image du volume

Il existe une correspondance entre la vitesse angulaire d'un accéléromètre thoracique et abdominal et la forme d'onde de l'effort ventilatoire globale respiratoire.

On peut donc prévoir une étape de calcul de la vitesse angulaire de chaque accéléromètre, ce qui permet une reconstruction d'une image du débit respiratoire, une reconstruction de la dynamique des mouvements respiratoires de l'individu.

A cet effet, on prévoit au moins l'une des combinaisons linéaires parmi :

- une combinaison linéaire de l'ACP du premier accéléromètre (thoracique) et de l'ACP du deuxième accéléromètre (abdominal) ; et
- une combinaison linéaire de la méthode angulaire appliquée au premier accéléromètre (thoracique) et de la méthode angulaire appliquée au deuxième accéléromètre (abdominal).

La figure 4 illustre de manière synchrone :

- le signal dPHI qui est la dérivée par rapport au temps du signal PHI de la figure 2,
- le signal dPRI qui est la dérivée par rapport au temps du signal PRI de la figure 2, et
- un signal FLOW de référence, qui est un signal de débit réel mesuré en l'espèce grâce à un masque respiratoire posé sur un individu.

La figure 4 illustre également clairement la pertinence de la présente invention puisque la le calcul de la dérivé n'amène pas de bruit particulier. Les 3 signaux dPHI, dPRI et FLOW sont globalement en phase. La présente invention permet de calculer une image du volume relatif, ainsi que sa variation en fonction du temps. En ce sens, elle est une PRI virtuelle.

Le premier accéléromètre et le deuxième accéléromètre fonctionnent comme un réseau de capteurs. Leurs mesures respectives sont synchronisées et correspondent respectivement à l'accélération du compartiment thoracique et du compartiment abdominal.

Il est alors possible de calculer les vitesses angulaires respectives. Ensuite, on peut comparer les variations de la vitesse angulaire du premier accéléromètre et les variations de la vitesse angulaire du deuxième accéléromètre sur une même base temporelle, et détecter par exemple si le premier accéléromètre et le deuxième accéléromètre sont en déphasage ou en opposition de phase et calculer par exemple un index de désynchronisation abdomino-thoracique.

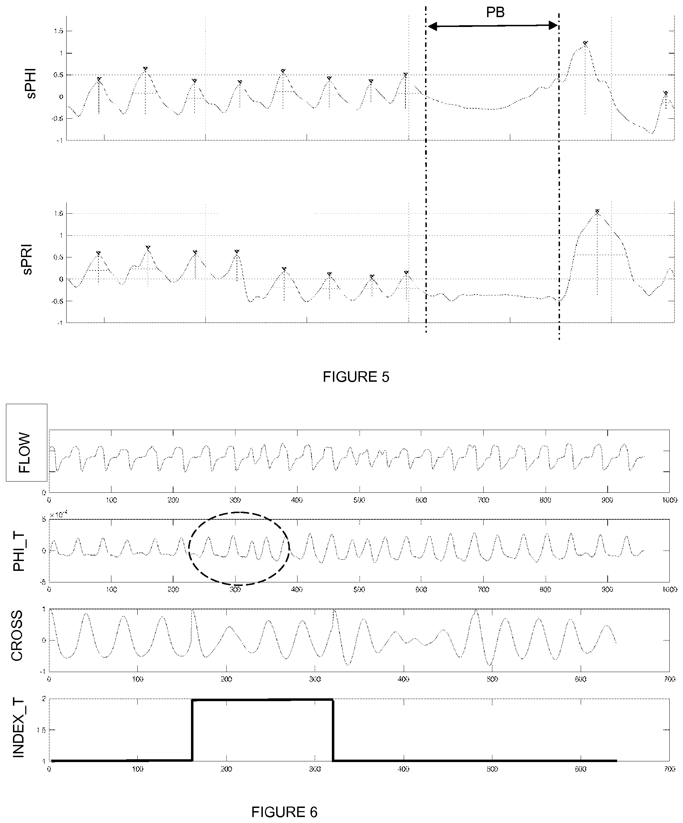

De manière similaire, la figure 5 illustre de manière synchrone un signal sPHI, qui est la somme des signaux PHI en l'espèce de la figure 1, et un signal sPRI, qui est la somme des signaux PRI en l'espèce de la figure 1.

Sur la figure 5, il est clair qu'une modification du rythme respiratoire (zone PB) est détectée par le procédé selon l'invention.

La présente invention permet de reconstruire une « image volume » des compartiments respiratoires et de suivre des évènements d'intérêt respiratoire. Elle présente une robustesse algorithmique et physiologique.

Comme exposé précédemment la première variante (ACP) et la deuxième variante (angulaire) sont combinables.

Par exemple, on peut prévoir dans une première phase de mettre en œuvre la première variante (ACP) sur une première fenêtre temporelle des mesures de l'un au moins des accéléromètres parmi le premier accéléromètre et le deuxième accéléromètre, puis de mettre en œuvre la deuxième variante (angulaire) sur une sous-fenêtre temporelle de la première fenêtre.

### Qualification de l'effort respiratoire global

Grâce à la présente invention, on peut, à l'aide d'un seul couple d'accéléromètres, qualifier le mode de fonctionnement ventilatoire d'un individu.

Dans une première variante, on prévoit de calculer une combinaison linéaire du premier vecteur final et du deuxième vecteur final. Par exemple, on prévoit des coefficients unité dans la combinaison linéaire.

Dans une deuxième variante, on prévoit de calculer au moins l'un des descripteurs parmi :

- une corrélation croisée normalisée sur au moins l'un des vecteurs finaux afin de caractériser la périodicité de l'effort respiratoire pour caractériser la périodicité (ou non périodicité) de l'un au moins des signaux parmi le signal issu du premier accéléromètre et le signal issu du deuxième accéléromètre,;

  o et par exemple comparer la périodicité (ou non périodicité) de l'un des deux signaux issu du premier accéléromètre et du deuxième accéléromètre par rapport à l'autre deux signaux issu du premier accéléromètre et du deuxième accéléromètre,

- la différence entre le premier vecteur final et le deuxième vecteur final, pour calculer au moins l'un parmi :

  o le déphasage éventuel le signal issu du premier accéléromètre et le signal issu du deuxième accéléromètre,
  o le couplage entre le signal issu du premier accéléromètre et le signal issu du deuxième accéléromètre par intercorrélation ou mutuelle information croisée,
  o un index de désynchronisation thoraco-abdominal,
  o et par exemple qualifier ainsi la cohérence du compartiment thoracique et du compartiment abdominal,

- une quantification de la contribution de chaque compartiment dans la ventilation globale.

### Instabilités

En outre, on peut prévoir une étape de détection d'instabilités, ou événements anormaux, par exemple non stationnaires, qui peuvent par exemple correspondre à un changement de position de l'individu et/ou de mouvement corporels, ce qui peut être le cas y compris la nuit.

Ainsi chaque signal issu du premier accéléromètre et du deuxième accéléromètre comprend des fenêtres temporelles de stabilité et éventuellement un ensemble d'au moins une fenêtre temporelle d'instabilité.

De préférence, on prévoit de mettre en œuvre les calculs décrits ci-avant dans une fenêtre de stabilité.

A cet effet, on peut prévoir de comparer la valeur du signal de l'un au moins des accéléromètres à une valeur seuil prédéterminée, et pour chaque fenêtre temporelle pour laquelle la valeur du signal est supérieure à la valeur seuil prédéterminée, alors les données de cette fenêtre temporelle ne sont pas prises en compte dans les calculs ; ainsi les calculs ne sont effectués que sur des phases stationnaires pendant lesquelles l'individu est immobile, ce qui améliore le rapport signal / bruit.

On peut alors lancer une nouvelle séquence d'étapes de prétraitement, filtrage, calculs de vecteurs finaux par ACP / méthode angulaire et qualification de l'effort respiratoire global.

Par exemple, la figure 6 et la figure 7 illustrent de manière synchrone :

- un signal FLOW de référence, qui est un signal de débit réel mesuré en l'espèce grâce à un masque respiratoire posé sur un individu,
- un signal PHI_T, qui est le signal PHI calculé en l'espèce selon la méthode angulaire, à partir des données brutes AX_T, AY_T et AZ_T issues du premier accéléromètre,

- un signal CROSS, qui est la corrélation croisée normalisée PHI_T,
- un index INDEX_T, qui est la caractérisation de la périodicité de l'effort ventilatoire thoracique reconstruit PHI_T, l'index INDEX_T étant un signal binaire pour lequel la valeur 1 correspond à un signal PHI_T périodique, et la valeur 2 à un signal PHI_T non périodique, obtenu en l'espèce par comparaison de la valeur du signal CROSS à une valeur seuil prédéterminée.

Sur la figure 6 et sur la figure 7, le signal PHI_T présente un défaut de périodicité, ce défaut étant cerclé en pointillés sur les figures 6 et 7 (observable également sur la référence FLOW).

Comme illustré par le signal INDEX_T, ce défaut de périodicité est clairement détecté par le procédé selon l'invention (front montant du signal INDEX_T sur la figure 6 et la figure 7).

Un retour à la normale, c'est à dire un signal PHI_T redevenant périodique est également détecté grâce au procédé selon l'invention, comme illustré par le front descendant du signal INDEX_T sur la figure 6.

## Revendications

1. Procédé de traitement de signal, comprenant des étapes consistant à :

   - enregistrer préalablement dans une mémoire un premier signal initial issu d'un premier ensemble d'au moins un accéléromètre (110) tridimensionnel positionné en position thoracique d'un individu,
   - enregistrer préalablement dans une mémoire un deuxième signal initial issu d'un deuxième ensemble d'au moins un accéléromètre (120) tridimensionnel positionné en position abdominale de l'individu, le deuxième signal étant synchronisé avec le premier signal,
   - traiter les données du premier signal initial pour calculer un premier vecteur final, qui est une reconstruction de l'effort respiratoire du compartiment thoracique et qui est représentatif des efforts thoraciques subis par le premier ensemble d'au moins un accéléromètre tridimensionnel,
   - traiter les données du deuxième signal initial pour calculer un deuxième vecteur final, qui est une reconstruction de l'effort respiratoire du compartiment abdominal et qui est représentatif des efforts abdominaux subis par le deuxième ensemble d'au moins un accéléromètre tridimensionnel,

   térisé en ce qu'il comprend en outre :

   - reconstruire des efforts respiratoires en fonction du premier vecteur final et du deuxième vecteur final, par :

     o un calcul angulaire, comprenant la mesure du mouvement angulaire de l'axe de rotation (r) de chaque accéléromètre, et le alcul

       ▪ du premier vecteur final, qui comprend les valeurs ($\phi_t$) de l'angle de rotation du premier ensemble d'au moins un accéléromètre (110) dans une fenêtre de temps, du deuxième vecteur final, qui comprend les valeurs ($\phi_t$) de l'angle de rotation du deuxième ensemble d'au moins un accéléromètre (120) dans une fenêtre de temps.

2. Procédé selon la revendication 1, dans lequel la reconstruction des efforts respiratoires en fonction du premier vecteur final et du deuxième vecteur final comprend en outre un calcul d'analyse en composantes principales, comprenant :

   - un premier vecteur final, qui est une reconstruction de l'effort respiratoire du compartiment thoracique, résultant d'une analyse en composantes principales sur une première matrice de départ correspondant aux données issues du premier accéléromètre, et
   - un deuxième vecteur final, qui est une reconstruction de l'effort respiratoire du compartiment abdominal, et résultant d'une analyse en composantes principales sur une deuxième matrice de départ correspondant aux données issues du deuxième accéléromètre

3. Procédé selon l'une quelconque des revendications précédentes, comprenant une étape consistant à qualifier le mode de fonctionnement ventilatoire dudit individu,

   o Soit par le calcul d'une combinaison linéaire du premier vecteur final et du deuxième vecteur final,

o Soit par le calcul d'au moins l'un des descripteurs parmi :

o **une corrélation** croisée normalisée sur au moins l'un des vecteurs finaux afin de caractériser la périodicité de l'effort respiratoire en caractérisant la périodicité de l'un au moins des signaux parmi le premier signal issu du premier accéléromètre et le deuxième signal issu du deuxième accéléromètre; et
o la différence entre le premier vecteur final et le deuxième vecteur final, pour calculer au moins l'un parmi :

• le déphasage et le couplage éventuel entre le premier signal issu du premier accéléromètre et le deuxième signal issu du deuxième accéléromètre, par intercorrélation ou mutuelle information croisée,
• un index de désynchronisation thoraco-abdominal,

o et qualifier ainsi la cohérence du compartiment thoracique et du compartiment abdominal,

o par une quantification de la contribution de chaque compartiment dans la ventilation globale.

4. Procédé selon l'une quelconque des revendications précédentes, dans lequel l'étape de traitement des données du premier signal initial, respectivement du deuxième signal initial, comprend une étape de détection d'une fenêtre d'instabilité temporelle dudit premier signal initial, respectivement deuxième signal initial et, en cas de détection d'une fenêtre d'instabilité temporelle, le procédé comprenant alors en outre une étape d'inhibition temporaire du calcul dudit premier vecteur final, respectivement dudit deuxième vecteur final, de préférence jusqu'à retrouver une fenêtre de stabilité temporelle.

5. Procédé selon l'une quelconque des revendications précédentes, dans lequel les données du premier signal initial, respectivement du deuxième signal, sont enregistrées sous forme d'une première matrice, respectivement d'une deuxième matrice ;

Et dans lequel au moins une des étapes parmi :

- traiter les données du premier signal initial et
- traiter les données du deuxième signal initial,

comprend une étape d'analyse en composantes principales de la première matrice, respectivement deuxième matrice, pour obtenir ledit premier vecteur final, respectivement ledit deuxième vecteur final ;
le procédé comprenant optionnellement en outre une étape de filtrage du premier vecteur final.

6. Procédé selon l'une quelconque des revendications précédentes, comprenant en outre une étape consistant à calculer, à chaque temps de mesure, l'angle de rotation par rapport à la gravité dudit premier ensemble d'au moins un accéléromètre tridimensionnel, respectivement du deuxième ensemble d'au moins un accéléromètre tridimensionnel, pour obtenir ledit premier vecteur final, respectivement ledit deuxième vecteur final.

7. Procédé selon l'une quelconque des revendications précédentes, comprenant une étape de filtrage passe-bande de l'un au moins parmi ledit premier signal et ledit deuxième signal, de préférence avec fréquence de coupure basse égale à 0,05 Hz et une fréquence de coupure haute égale à 0,8 Hz ; et optionnellement avec un filtre à réponse impulsionnelle réelle.

8. Procédé selon l'une quelconque des revendications précédentes, dans lequel l'étape de méthode angulaire consiste à :

- calculer l'angle de rotation de chaque accéléromètre par rapport à la gravité à chaque temps de mesure; et pour chaque accéléromètre :
- mesurer et normaliser le vecteur accélération (a),
- mesurer le mouvement angulaire de l'axe de rotation (r) dudit accéléromètre,
- calculer l'angle de rotation ($\theta$t) et l'axe de rotation (rt) du vecteur accélération (a) entre deux mesures consécutives au temps t-1 et au temps t par le produit scalaire et le

$$\theta_t = \cos^{-1}(\boldsymbol{a}_t \cdot \boldsymbol{a}_{t-1})$$

$$r_t = a_t \times a_{t-1}$$

produit vectoriel des deux vecteurs :

- normaliser la direction de l'axe de rotation (r) dans un hémisphère prédéterminé par comparaison à un axe de référence ($r_{ref}$) et calculer la valeur de :

$$r_t' = \begin{cases} r_t, & r_t \cdot r_{ref} \geq 0 \\ -r_t, & r_t \cdot r_{ref} < 0 \end{cases}$$

- calculer $r_t$ l'axe de rotation (r) normalisé moyenné sur une fenêtre de temps de durée (W) tel que :

$$\bar{r}_t = \text{normalise} \left( \sum_{i=-W/2}^{W/2} H(i)\theta_{t+i} r_{t+i}' \right)$$

avec H(n) une fenêtre prédéterminée ;
et de manière similaire,

- déterminer $\bar{a}_t$ l'accélération moyenne normalisée moyennée sur la même fenêtre de temps de durée (W), tel que

$$\bar{a}_t = \text{normalise} \left( \sum_{i=-W/2}^{W/2} a_{t+i} \right)$$

- calculer $\phi_t$ l'angle de rotation dudit accéléromètre tel que

$$\phi_t = \sin^{-1}((\bar{a}_t \times \bar{r}_t) \cdot a_t)$$

$$\omega_t = \frac{d\phi}{dt}$$

- **calculer,** si nécessaire, la vitesse angulaire ωt telle que
- enregistrer les valeurs de $\phi_t$ sous forme de vecteur final.

9. Procédé selon la revendication 8, comprenant des étapes consistant à :

- calculer un index (INDEX_T), qui est un signal binaire pour lequel la valeur 1 correspond à un signal (PHI_T) périodique, et la valeur 2 à un signal (PHI_T) non périodique, obtenu par comparaison de la valeur du signal (CROSS) à une valeur seuil prédéterminée et qui est la caractérisation de la périodicité de l'effort ventilatoire thoracique reconstruit (PHI_T),

où :

- le signal (PHI_T) est le signal (PHI) calculé selon la méthode angulaire, à partir des données brutes issues du premier accéléromètre
- le signal (PHI) est calculé selon la méthode angulaire, à partir des données brutes issues du premier accéléromètre et des données brutes issues du deuxième accéléromètre,
- le signal (CROSS) est la corrélation croisée normalisée du signal (PHI_T).

10. Procédé selon l'une quelconque des revendications 8 à 9, comprenant une étape consistant à calculer au moins l'un des descripteurs parmi :

- le déphasage éventuel entre le signal issu du premier ensemble d'au moins un accéléromètre et le signal issu du deuxième ensemble d'au moins un accéléromètre,
- le couplage entre le signal issu du premier ensemble d'au moins un accéléromètre et le signal issu du deuxième ensemble d'au moins un accéléromètre par intercorrélation ou mutuelle information croisée, et
- un index de désynchronisation thoraco-abdominal.

11. Procédé selon l'une quelconque des revendications précédentes, comprenant en outre une étape de détection de changement de position de l'individu.

**Patentansprüche**

1. Signalverarbeitungsverfahren, umfassend die folgenden Schritte:

   - vorheriges Aufzeichnen eines ersten Anfangssignals, das aus einem ersten Satz von mindestens einem dreidimensionalen Beschleunigungsmesser (110) stammt, der in der Thoraxposition einer Person positioniert ist, in einem Speicher,
   - vorheriges Aufzeichnen eines zweiten Anfangssignals, das aus einem zweiten Satz von mindestens einem dreidimensionalen Beschleunigungsmesser (120) stammt, der in der Abdomenposition der Person positioniert ist, in einem Speicher, wobei das zweite Signal mit dem ersten Signal synchronisiert ist,
   - Verarbeiten der Daten des ersten Anfangssignals, um einen ersten Endvektor zu berechnen, der eine Rekonstruktion der Atembewegung des Thoraxraums ist und repräsentativ für die Thoraxbewegungen ist, die von dem ersten Satz von mindestens einem dreidimensionalen Beschleunigungsmesser erfahren werden,
   - Verarbeiten der Daten des zweiten Anfangssignals, um einen zweiten Endvektor zu berechnen, der eine Rekonstruktion der Atembewegung des Abdominalraums ist und repräsentativ für die Abdomenbewegungen ist, die von dem zweiten Satz von mindestens einem dreidimensionalen Beschleunigungsmesser erfahren werden,

   **dadurch gekennzeichnet, dass** es ferner umfasst:

   - Rekonstruieren der Atembewegungen in Abhängigkeit des ersten Endvektors und des zweiten Endvektors durch:

     o eine Winkelberechnung, die die Messung der Winkelbewegung der Rotationsachse (r) jedes Beschleunigungsmessers umfasst, und die Berechnung

       ▪ des ersten Endvektors, der die Werte ($\Phi_t$) des Rotationswinkels des ersten Satzes von mindestens einem Beschleunigungsmesser (110) in einem Zeitfenster umfasst,
       ▪ des zweiten Endvektors, der die Werte ($\Phi_t$) des Drehwinkels des zweiten Satzes von mindestens einem Beschleunigungsmesser (120) in einem Zeitfenster umfasst.

2. Verfahren nach Anspruch 1, bei dem die Rekonstruktion der Atembewegungen in Abhängigkeit von dem ersten Endvektor und von dem zweiten Endvektor außerdem eine Berechnung zur Analyse der Hauptkomponenten umfasst, umfassend:

   - einen ersten Endvektor, der eine Rekonstruktion der Atembewegung des Thoraxraums ist, die sich aus einer Analyse der Hauptkomponenten einer ersten Ausgangsmatrix ergibt, die den Daten entspricht, die aus dem ersten Beschleunigungsmesser stammen, und
   - einen zweiten Endvektor, der eine Rekonstruktion der Atembewegung des Abdominalraums ist, und die sich aus einer Analyse der Hauptkomponenten einer zweiten Ausgangsmatrix ergibt, die den Daten entspricht, die aus dem zweiten Beschleunigungsmesser stammen.

3. Verfahren nach einem der vorhergehenden Ansprüche, umfassend einen Schritt, der darin besteht, die Art der Beatmung der Person zu beurteilen,

   o entweder durch Berechnung einer linearen Kombination des ersten Endvektors und des zweiten Endvektors,
   ∘ oder durch Berechnung mindestens eines der Deskriptoren aus:

     ∘ einer normierten Kreuzkorrelation auf mindestens einem der Endvektoren, um die Periodizität der Atem-

13

bewegung zu charakterisieren, indem die Periodizität mindestens eines der Signale entweder des ersten Signals, das aus dem ersten Beschleunigungsmesser stammt, oder des zweiten Signals, das aus dem zweiten Beschleunigungsmesser stammt, charakterisiert wird; und

◦ der Differenz zwischen dem ersten Endvektor und dem zweiten Endvektor, um mindestens eines von Folgendem zu berechnen:

• die Phasenverschiebung und die mögliche Kopplung zwischen dem ersten Signal, das aus dem ersten Beschleunigungsmesser stammt, und dem zweiten Signal, das aus dem zweiten Beschleunigungsmesser stammt, durch Kreuzkorrelation oder gegenseitige Kreuzinformation,
• einen Index der thorakoabdominalen Desynchronisation,

◦ und um somit die Kohärenz des Thorax- und des Abdominalraums zu beurteilen,
◦ durch eine Quantifizierung des Beitrags jedes Raums zu der Gesamtbeatmung.

4. Verfahren nach einem der vorhergehenden Ansprüche, bei dem der Schritt der Verarbeitung der Daten des ersten Anfangssignals oder des zweiten Anfangssignals einen Schritt der Erfassung eines Fensters zeitlicher Instabilität des ersten Anfangssignals oder des zweiten Anfangssignals umfasst und das Verfahren im Falle der Erfassung eines Fensters zeitlicher Instabilität dann außerdem einen Schritt der vorübergehenden Hemmung der Berechnung des ersten Endvektors oder des zweiten Endvektors umfasst, vorzugsweise bis ein Fenster zeitlicher Stabilität wiedererlangt wird.

5. Verfahren nach einem der vorhergehenden Ansprüche, bei dem die Daten des ersten Anfangssignals oder des zweiten Signals in Form einer ersten Matrix oder einer zweiten Matrix aufgezeichnet werden;

Und bei dem mindestens einer der folgenden Schritte:

- Verarbeiten der Daten des ersten Anfangssignals und
- Verarbeiten der Daten des zweiten Anfangssignals,

einen Schritt der Analyse der Hauptkomponenten der ersten Matrix oder der zweiten Matrix umfasst, um den ersten Endvektor oder den zweiten Endvektor zu erhalten;
wobei das Verfahren optional außerdem einen Schritt des Filterns des ersten Endvektors umfasst.

6. Verfahren nach einem der vorhergehenden Ansprüche, das außerdem einen Schritt umfasst, der darin besteht, bei jeder Messzeit den Drehwinkel in Bezug auf die Schwerkraft des ersten Satzes von mindestens einem dreidimensionalen Beschleunigungsmesser oder des zweiten Satzes von mindestens einem dreidimensionalen Beschleunigungsmesser zu berechnen, um den ersten Endvektor oder den zweiten Endvektor zu erhalten.

7. Verfahren nach einem der vorhergehenden Ansprüche, umfassend einen Bandpassfilterungsschritt von mindestens einem des ersten Signals und des zweiten Signals, vorzugsweise mit einer unteren Grenzfrequenz von 0,05 Hz und einer oberen Grenzfrequenz von 0,8 Hz; und optional mit einem Filter mit realer Impulsantwort.

8. Verfahren nach einem der vorhergehenden Ansprüche, bei dem der Winkelmethodenschritt darin besteht:

- den Rotationswinkel jedes Beschleunigungsmessers in Bezug auf die Schwerkraft bei jedem Messzeitpunkt zu berechnen; und für jeden Beschleunigungsmesser:
- den Beschleunigungsvektor (a) zu messen und zu normieren,
- die Winkelbewegung der Rotationsachse (r) des Beschleunigungsmessers zu messen,
- den Rotationswinkel ($\theta$t) und die Rotationsachse (rt) des Beschleunigungsvektors (a) zwischen zwei aufeinanderfolgenden Messungen zu der Zeit t-1 und zu der Zeit t durch das Skalarprodukt und das

$$\theta_t = \cos^{-1}(\boldsymbol{a}_t \cdot \boldsymbol{a}_{t-1})$$

$$\boldsymbol{r}_t = \boldsymbol{a}_t \times \boldsymbol{a}_{t-1}$$

Vektorprodukt der beiden Vektoren: zu berechnen,
- die Richtung der Rotationsachse (r) in einer vorbestimmten Hemisphäre durch Vergleich mit einer Referenzachse ($r_{ref}$) zu normieren und den Wert zu berechnen von:

$$r'_t = \begin{cases} \boldsymbol{r}_t, & \boldsymbol{r}_t \cdot \boldsymbol{r}_{ref} \geq 0 \\ -\boldsymbol{r}_t, & \boldsymbol{r}_t \cdot \boldsymbol{r}_{ref} < 0 \end{cases}$$

- Berechnen von $\bar{r}_t$, der normierten, über ein Zeitfenster der Dauer (W) gemittelten Rotationsachse (r), wie folgt:

$$\bar{\boldsymbol{r}}_t = \text{normiert} \left( \sum_{i=-W/2}^{W/2} H(i)\theta_{t+i}\boldsymbol{r}'_{t+i} \right)$$

wobei H(n) ein vorbestimmtes Fenster ist;
und in ähnlicher Weise,

- Bestimmen von $\bar{a}_t$, der gemittelten normierten Beschleunigung in dem gleichen Zeitfenster der Dauer (W), wie folgt

$$\bar{\boldsymbol{a}}_t = \text{normiert} \left( \sum_{i=-W/2}^{W/2} a_{t+i} \right)$$

- Berechnen von $\Phi_t$, dem Rotationswinkel des Beschleunigungsmessers, wie

$$\phi_t = \sin^{-1}\big((\bar{\boldsymbol{a}}_t \times \bar{\boldsymbol{r}}_t) \cdot \boldsymbol{a}_t\big)$$

folgt

$$\omega_t = \frac{\mathrm{d}\phi}{\mathrm{d}t}$$

- falls erforderlich, Berechnen der Winkelgeschwindigkeit $\omega$t, wie folgt
- Aufzeichnen der Werte von $\Phi_t$ in Form eines Endvektors.

9. Verfahren nach Anspruch 8, umfassend die Schritte, die aus Folgendem bestehen:

- Berechnen eines Index (INDEX_T), der ein Binärsignal ist, bei dem der Wert 1 einem periodischen Signal (PHI_T) und der Wert 2 einem nicht periodischen Signal (PHI_T) entspricht, das durch Vergleich des Werts des Signals (CROSS) mit einem vorbestimmten Schwellenwert erhalten wird und das die Beurteilung der Periodizität der rekonstruierten Thoraxbeatmungsbewegung (PHI_T) ist,

wobei:

- das Signal (PHI_T) das nach der Winkelmethode aus den Rohdaten des ersten Beschleunigungsmessers berechnete Signal (PHI) ist,
- das Signal (PHI) nach der Winkelmethode aus den Rohdaten, die aus dem ersten Beschleunigungsmesser stammen, und den Rohdaten, die aus dem zweiten Beschleunigungsmesser stammen, berechnet wird;
- das Signal (CROSS) die normierte Kreuzkorrelation des Signals (PHI_T) ist.

10. Verfahren nach einem der Ansprüche 8 bis 9, umfassend einen Schritt, der darin besteht, mindestens einen der Deskriptoren zu berechnen aus:

- der etwaigen Phasenverschiebung zwischen dem Signal, das aus dem ersten Satz von mindestens einem Beschleunigungsmesser stammt, und dem Signal, das aus dem zweiten Satz von mindestens einem Beschleunigungsmesser stammt,
- der Kopplung zwischen dem Signal, das aus dem ersten Satz von mindestens einem Beschleunigungsmesser stammt, und dem Signal, das aus dem zweiten Satz von mindestens einem Beschleunigungsmesser stammt, durch Kreuzkorrelation oder gegenseitige Kreuzinformation, und
- einem Index der thorakoabdominalen Desynchronisation.

11. Verfahren nach einem der vorhergehenden Ansprüche, das außerdem einen Schritt zur Erfassung einer Positionsänderung der Person umfasst.

**Claims**

1. **Signal-processing** method, comprising steps consisting in:

   - storing beforehand in a memory a first initial signal generated by a first set of at least one three-axis accelerometer (110) positioned on the thorax of an individual,
   - storing beforehand in a memory a second initial signal generated by a second set of at least one three-axis accelerometer (120) positioned on the abdomen of the individual, the second signal being synchronized with the first signal,
   - processing the data of the first initial signal to compute a first final vector, which is a reconstruction of the respiratory effort of the thoracic compartment, and which is representative of the thoracic efforts undergone by the first set of at least one three-axis accelerometer,
   - processing the data of the second initial signal to compute a second final vector, which is a reconstruction of the respiratory effort of the abdominal compartment, and which is representative of the abdominal efforts undergone by the second set of at least one three-axis accelerometer,

   **characterized in that** it further comprises:

   - reconstructing the respiratory efforts based on the first final vector and on the second final vector via:

     ◦ an angular computation, comprising measuring the angular movement of the rotation axis (r) of each accelerometer, and computing

       ▪ the first final vector, which contains the values ($\Phi_t$) of the rotation angle of the first set of at least one accelerometer (110) in a time window,
       ▪ the second final vector, which contains the values ($\Phi_t$) of the rotation angle of the second set of at least one accelerometer (120) in a time window.

2. Method according to Claim 1, wherein reconstructing the respiratory efforts based on the first final vector and on the second final vector further comprises a principal component analysis, comprising:

   - a first final vector, which is a reconstruction of the respiratory effort of the thoracic compartment, resulting from a principal component analysis of a first starting matrix corresponding to the data generated by the first accelerometer, and
   - a second final vector, which is a reconstruction of the respiratory effort of the abdominal compartment, and resulting from a principal component analysis of a second starting matrix corresponding to the data generated by the second accelerometer.

3. Method according to either of the preceding claims, comprising a step consisting in qualifying the breathing mode of operation of said individual,

   ◦ **either** by computing a linear combination of the first final vector and of the second final vector,
   ◦ or by computing at least one of the descriptors among:

     ◦ a normalized cross-correlation of at least one of the final vectors, in order to characterize the periodicity of the respiratory effort with a view to characterizing the periodicity of at least one of the signals among the first signal generated by the first accelerometer and the second signal generated by the second accelerometer; and

o the difference between the first final vector and the second final vector, to compute at least one among:

• any phase shift and coupling between the first signal generated by the first accelerometer and the second signal generated by the second accelerometer, as given by intercorrelation or mutual cross-information,
• an index of thoracic-abdominal desynchronization,

o and to thus qualify the coherence of the thoracic compartment and of the abdominal compartment,
o via a quantification of the contribution of each compartment to the overall breath.

4. Method according to any of the preceding claims, wherein the step of processing the data of the first initial signal and the step of processing the data of the second initial signal comprise a step of detecting a time window of instability in said first initial signal and a step of detecting a time window of instability in said second initial signal, and, in case of detection of a time window of instability, the method then furthermore comprises a step of temporarily inhibiting the computation of said first final vector and a step of temporarily inhibiting the computation of said second final vector, preferably until a time window of stability is regained, respectively.

5. Method according to any of the preceding claims, wherein the data of the first initial signal and the data of the second signal are stored in the form of a first matrix and in the form of a second matrix, respectively;

and wherein at least one of the steps among:

- processing the data of the first initial signal, and
- processing the data of the second initial signal,

comprises a step of principal component analysis of the first matrix and a step of principal component analysis of the second matrix to obtain said first final vector and to obtain said second final vector, respectively; the method optionally furthermore comprising a step of filtering the first final vector.

6. Method according to any of the preceding claims, furthermore comprising a step consisting in computing, at each measurement time, the rotation angle with respect to gravity of said first set of at least one three-axis accelerometer and the rotation angle with respect to gravity of said second set of at least one three-axis accelerometer to obtain said first final vector and to obtain said second final vector, respectively.

7. Method according to any of the preceding claims, comprising a step of applying a band-pass filter to at least one among said first signal and said second signal, said band-pass filter preferably having a lower cut-off frequency equal to 0.05 Hz and an upper cut-off frequency equal to 0.8 Hz, and optionally being a finite-impulse-response filter.

8. Method according to any of the preceding claims, wherein the angular method step consists in:

- computing the rotation angle of each accelerometer with respect to gravity at each measurement time; and for each accelerometer:
- measuring and normalizing the acceleration vector (a),
- measuring the angular movement of the rotation axis (r) of said accelerometer,
- computing the rotation angle ($\theta t$) and the rotation axis (rt) of the acceleration vector (a) between two consecutive measurements at time t-1 and at time t by means of the scalar product and the

$$\theta_t = \cos^{-1}(\boldsymbol{a}_t \cdot \boldsymbol{a}_{t-1})$$

$$\boldsymbol{r}_t = \boldsymbol{a}_t \times \boldsymbol{a}_{t-1}$$

cross product of the following two vectors:
- normalizing the direction of the rotation axis (r) in a preset hemisphere, by comparison with a reference axis ($r_{ref}$) and computing the value of:

$$r'_t = \begin{cases} r_t, & r_t \cdot r_{ref} \geq 0 \\ -r_t, & r_t \cdot r_{ref} < 0 \end{cases}$$

- computing $\bar{r}_t$ the normalised rotation axis (r) averaged over a time window of duration (W) such that:

$$\bar{r}_t = \text{normalize} \left( \sum_{i=-W/2}^{W/2} H(i)\theta_{t+i}r'_{t+i} \right)$$

with H(n) a preset window;
and similarly,

- determining $\bar{a}_t$ the normalized average acceleration averaged over the same time window of duration (W), such that

$$\bar{a}_t = \text{normalize} \left( \sum_{i=-W/2}^{W/2} a_{t+i} \right)$$

- **computing** $\Phi_t$ the rotation angle of said accelerometer such that

$$\phi_t = \sin^{-1}\big((\bar{a}_t \times \bar{r}_t) \cdot a_t\big)$$

$$\omega_t = \frac{d\phi}{dt}$$

- computing, if necessary, the angular velocity ωt such that
- recording the values of $\Phi_t$ in the form of a final vector.

9. Method according to Claim 8, comprising steps consisting in:

- computing an index (INDEX_T), which is a binary signal for which the value 1 corresponds to a periodic signal (PHI_T) and the value 2 to a non-periodic signal (PHI_T), which is obtained by comparing the value of the signal (CROSS) with a preset threshold value, and which is the characterization of the periodicity of the reconstructed thoracic breathing effort (PHI_T),

wherein:

- the signal (PHI_T) is the signal (PHI) computed using the angular method, from the raw data generated by the first accelerometer,
- the signal (PHI) is computed using the angular method, from the raw data generated by the first accelerometer and from the raw data generated by the second accelerometer;
- the signal (CROSS) is the normalized cross-correlation of the signal (PHI_T).

10. Method according to either of Claims 8 and 9, comprising a step consisting in computing at least one of the descriptors among:

- any phase shift between the signal generated by the first set of at least one accelerometer and the signal generated by the second set of at least one accelerometer,
- the coupling between the signal generated by the first set of at least one accelerometer and the signal generated by the second set of at least one accelerometer, as given by intercorrelation or mutual cross-information, and

- an index of thoracic-abdominal desynchronization.

11. Method according to any of the preceding claims, furthermore comprising a step of detecting a change in position of the individual.

FIGURE 1

FIGURE 2

FIGURE 3

FIGURE 4

FIGURE 5

FIGURE 6

FIGURE 7

FIGURE 8A

FIGURE 8B

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Littérature non-brevet citée dans la description**

- **ATENA ROSHAN FEKR et al.** Respiration Disorders Classification With Informative Features for m-Health Applications. *IEEE JOURNAL OF BIOMEDICAL AND HEALTH INFORMATICS*, 21 July 2015, vol. 20 (3), 733-747